# EUROPEAN PATENT APPLICATION

(11) **EP 3 431 058 A1**
(43) Date of publication of application: **23.01.2019**
(21) Application number: 18182959.9
(22) Date of filing: 11.07.2018
(51) Int. Cl.: A61F 5/451, B65D 81/32

(54) **PORTABLE WASTE CONTAINMENT UNIT**

(30) Priority: 17.07.2017 US 201715651618
(71) Applicant: Baymen Global Company Limited, Belize City (BZ)
(72) Inventor: Del Cid, Oliver Darien, Belize City (BZ)
(74) Representative: Sandri, Sandro

(57) **Abstract**

One embodiment provides a portable waste containment apparatus, including: a main body (100); the main body (100) comprising one or more sealable containers (101A and 101B), each of the one or more sealable containers (101A and 101B) having an inner diameter; the main body (100) comprising one or more inner walls segregating the one or more sealable containers (101A and 101B) from each of the other one or more sealable containers (101A and 101B); and one or more lids (102A and 102B) for each of the one of more sealable containers (101A and 10IB), wherein the one or more lids (102A and 102B) seal the corresponding one or more sealable containers (101A and 101B). Other aspects are described and claimed.

## Description

### BACKGROUND

Outdoor enthusiasts face challenges when visiting environmentally sensitive areas. One of these challenges is the proper disposal of waste in the form of excrement. Many natural areas have a policy of visitors packing this waste out of the area. Outdoor enthusiasts often use the "wag bag". The wag bag is a rudimentary system of carrying waste out of the area in a plastic bag. Wag bags often fail with respect to structural integrity leaving a mess, smell, and non-hygienic solution to the problem.

### BRIEF SUMMARY

In summary, one aspect provides a portable waste containment apparatus, comprising: a main body; the main body comprising one or more sealable containers, each of the one or more sealable containers having an inner diameter; the main body comprising one or more inner walls segregating the one or more sealable containers from each of the other one or more sealable containers; and one or more lids for each of the one of more sealable containers, wherein the one or more lids seal the corresponding one or more sealable containers.

Another aspect provides a waste management product, comprising: a main body; wherein the main body comprises a first sealable container having a first inner diameter and a second sealable container having a second inner diameter, wherein the first inner diameter is different from the second inner diameter; the first sealable container having a corresponding first lid wherein the first lid comprises one or more threads that interact with threads of the first sealable container for connecting the first lid to the first sealable container; the second sealable container having a corresponding second lid wherein the second lid comprises one or more threads that interact with threads of the second sealable container for connecting the second lid to the second sealable container; and one or more handles disposed at a portion of the main body.

A further aspect provides a waste management product, comprising: a main body comprising plastic, wherein the main body comprises at least one handle and at least one recessed area and wherein the main body comprises two sealable containers internally separated from each other via an inner wall; wherein a first of the two sealable containers is aligned at an angle with respect to the second of the two sealable containers; and two lids, wherein each of the two lids correspond to one of the two sealable containers and wherein each of the two lids are connectable to and seal the corresponding sealable container; each of the two lids comprising at least two layers, wherein at least one of the at least two layers comprises plastic and wherein at least another of the at least two layers comprises a soft molded material; each of the two lids comprising a tether attachable to the corresponding sealable container.

The foregoing is a summary and thus may contain simplifications, generalizations, and omissions of detail; consequently, those skilled in the art will appreciate that the summary is illustrative only and is not intended to be in any way limiting.

For a better understanding of the embodiments, together with other and further features and advantages thereof, reference is made to the following description, taken in conjunction with the accompanying drawings. The scope of the invention will be pointed out in the appended claims.

### BRIEF DESCRIPTION OF THE SEVERAL VIEWS OF THE DRAWINGS

FIG. 1 illustrates a front view of an embodiment.
FIG. 2 illustrates a side view of an embodiment.
FIG. 3 illustrates a rear view of an embodiment.
FIG. 4 illustrates a top view of an embodiment.
FIG. 5 illustrates a bottom view of a front and a side of an embodiment.
FIG. 6 illustrates a top view of a front and a side of an embodiment.

### DETAILED DESCRIPTION

It will be readily understood that the components of the embodiments, as generally described and illustrated in the figures herein, may be arranged and designed in a wide variety of different configurations in addition to the described example embodiments. Thus, the following more detailed description of the example embodiments, as represented in the figures, is not intended to limit the scope of the embodiments, as claimed, but is merely representative of example embodiments.

Reference throughout this specification to "one embodiment" or "an embodiment" (or the like) means that a particular feature, structure, or characteristic described in connection with the embodiment is included in at least one embodiment. Thus, the appearance of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, et cetera. In other instances, well known structures, materials, or operations are not shown or described in detail to avoid obfuscation.

Outdoor enthusiasts face challenges when visiting environmentally sensitive areas, including the proper disposal of waste in the form of excrement. With the increasing number of natural areas with policies of packing waste out of the area, and voluntary removal by some enthusiasts, the need for a safe and effective way of carrying this waste back to a proper disposal site is increasing.

These outdoor enthusiasts often use the "wag bag". The "WAG" in wag bag is an acronym for "Waste Alleviation & Gelling". The wag bag is a rudimentary system of carrying waste out of the area in a plastic bag. Wag bag leakage creates a mess, smell, and non-hygienic solution to the problem. Additionally, improperly stored waste at a campsite may pose a safety risk and cause nuisance issues since wild animals may be attracted to the waste.

The existing wag bags may be a simple plastic bag, or a bag with a sealable closure. The wag bags may contain preloaded material to lessen the odor through fragrances. The preloaded material is generally a gel or a powder. The preloaded material attempts to gel or solidify liquids in the waste to reduce spills. The preloaded material may also contain a decay catalyst to accelerate waste decay.

Wag bags may be used in conjunction with a device that serves as a support structure to keep the wag bag open and positioned during use. These structures are made of cardboard, wire, plastic, or combination thereof. Traditional wag bag usage creates additional trash destined for a landfill. The wag bag, excrement, and associated chemicals contained inside the bag, are placed in a municipal trash receptacle upon returning from an outdoor trip. The waste and material from the bags and/or support structure take up landfill space circumventing proper sewage treatment and recycling facilities.

Accordingly, an embodiment provides a reusable, sealable container to contain human waste when proper bathroom facilities are not available. The container may have a screw top lid with a retention strap. The container may have a plurality of chambers with a plurality of lids. The container is a hygienic, mess free way to pack waste out of an area without restroom facilities or where waste may not be left in the environment. The container also greatly reduces landfill bound trash, due to the ability to clean and reuse the container. The entire container may be biodegradable, for example, in case the container is dropped to an inaccessible area or accidentally forgotten. The product as described herein may be desirable to mountain climbers, rock climbers, hikers, campers, skiers, snowboards, spelunkers, as well as any outdoor enthusiast who is required or otherwise wishes to contain his or her fecal waste for subsequent disposal.

Additionally, because the container is water tight, the container can safeguard any object from weather or water. Accordingly, the container as described herein may be used, rather than as a waste management product, to store other items such as satellite phones, cell phones, GPS units, electronics, fire starting apparatus, toilet paper, documents, and the like. Additionally, the materials of the container may provide for mechanisms for advertisement or personalization. As an example, the lids may include a soft overmolding which can be laser etched with words, logos, characters, or anything else that a person may want to include. As another example, the container may include a recessed area that is ideal for placing a sticker or label which may be personalized or include an advertisement.

The illustrated example embodiments will be best understood by reference to the figures. The following description is intended only by way of example, and simply illustrates certain example embodiments.

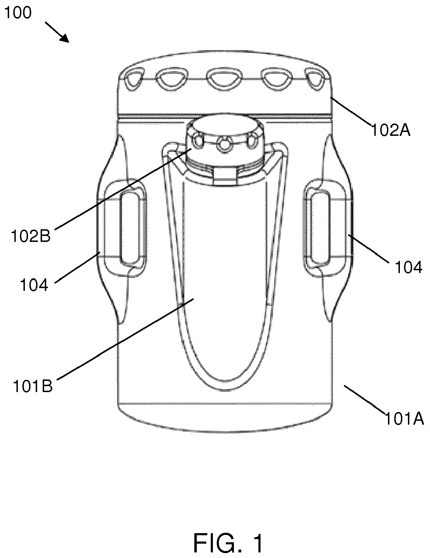

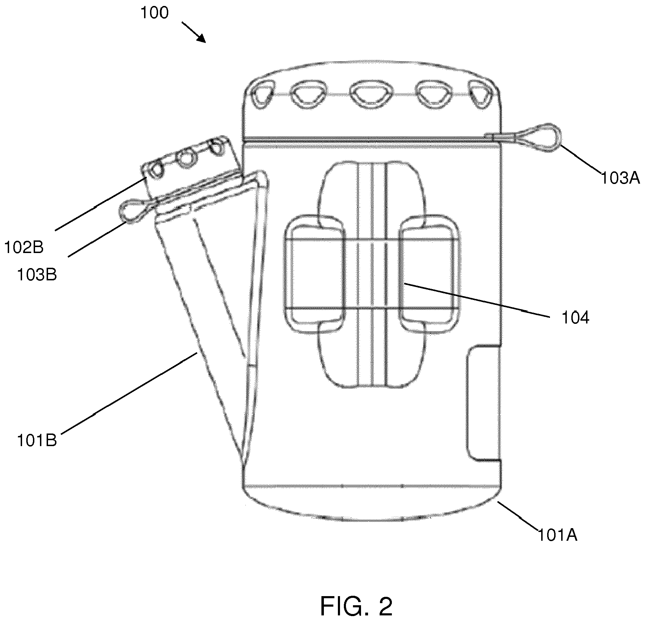

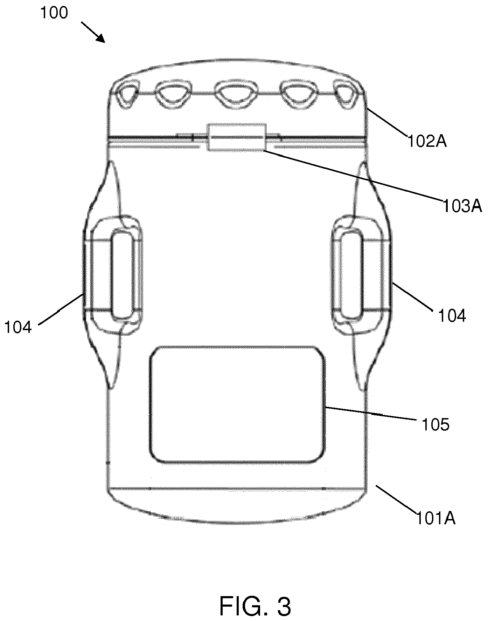

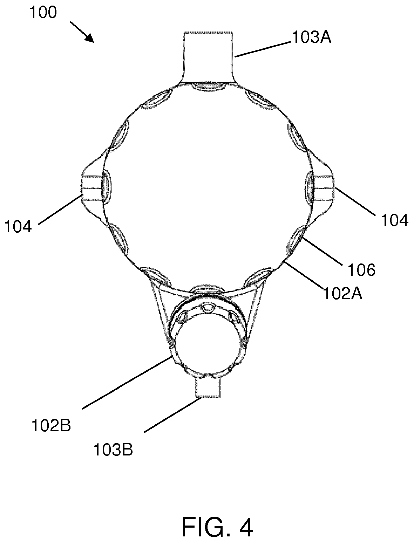

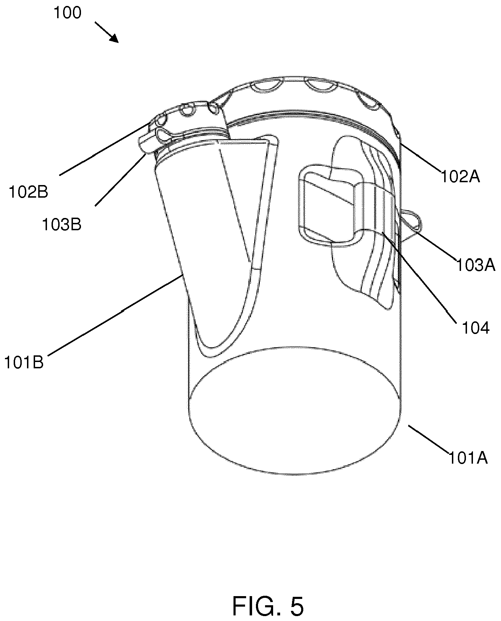

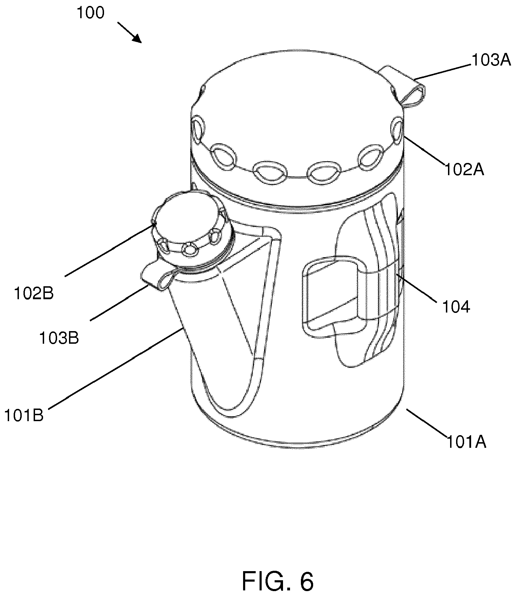

FIGs. 1 - 6 illustrate different views of an embodiment. FIG. 1 illustrates a front view, FIG. 2 illustrates a side view, FIG. 3 illustrates a back view, FIG. 4 illustrates a top view, FIG. 5 illustrates a front view from a bottom angle, and FIG. 6 illustrates a front view from a top angle. Any dimensions discussed are for reference purposes only. The container 100 can be designed to be any size and may be smaller or larger or have different proportions than those described. Additionally, the figures show two sealable containers, but embodiments may include only a single container or may include more than two containers. In one embodiment the main body 100 is made of or has a one or more separate compartments or sealable containers 101A and 101B. Each of the compartments or sealable containers may be internally separated from the other sealable containers by an internal wall. This allows for objects, including fecal matter placed in one of the containers to be held separate from objects in the other containers. Additionally, because the objects are held separate from each other, objects in one compartment cannot contaminate objects in the other compartments.

The one or more separate compartments 101A and 101B may be cylindrical or circular in cross section, which may allow for an easier sealing arrangement, for example, using threads, snap on, twist locks, latching mechanisms, or other sealing mechanisms. However, the containers 101A and 101B may be other shapes, for example, squares, rectangles, octagons, and the like. The one or more compartments 101A and 101B may be of different diameters or of the same diameter. In one embodiment the one or more separate compartments 101A and 101B may meet at an angle forming a "V" shape. In other words, one or more of the sealable containers 101A and 101B may be at substantially an angle with respect to one or more other sealable containers. Alternatively, the one or more separate compartments 101A and 101B may be parallel or substantially parallel to other compartments.

One or more of the compartments 101A and 101B may include a corresponding lid 102A and 102B, respectively. Not all compartments have to have a corresponding lid. Closure of the lid may via a threaded, compression sealed, twist lock, snap on, or the like, type of closure. Alternatively, an embodiment may include additional latch, catch, or snap mechanisms, for example, mechanisms attached to either the lid or container that are movable and engages with a corresponding portion of the lid or container. The lid may have an o-ring seal that is disposed in a position that when the lid is coupled to the compartment the o-ring is disposed between the lid and the compartment. A lid and the corresponding container may have complimentary surfaces to achieve a seal. The lid may be made of the same plastic as the main body. Alternatively, the lid may be of a different material as the main body.

An embodiment may include one or more tethers 103A and 103B that may be used to tether the lid 102A and 102B to the corresponding container 101A and 102A and retain the lid to the entire body of the product 100. In one embodiment, the tether or tethering strap 103A and 103B may be a part of the lid. For example, the tether 103A and 103B may be injection molded as part of the lid 102A and 102B. The tethering strap's 103A and 103B main function is to prevent the loss of a lid 102A and 102B from the main body 101A and 101B. The tethering strap 103A and 103B may be made of a pliable plastic, compostable material, webbing, stranded material, or the like. The tethering strap 103A and 103B may be affixed by a collar on the main body end of the tether over a portion of the compartment. Alternatively, the tether 103A and 103B may attach to the main body using a stud, ring, fastener, or the like. The tethering strap 103A and 103B may be affixed to the lid 102A and 102B by a collar, a stud on the lid, or any fastening means. Tethers 103A and 103B may be included for every lid/compartment pair, for only some of the lid/compartment pairs, or may not be included at all.

The main body 100 may have one or more handles 104 on the side. The one or more handles 104 may be used for holding the main body 100 by a user, or for attaching the main body 100 to another piece of equipment. For example, a carabiner, strap, rope, or the like, may be run through the aperture formed by the handle 104 and the outer wall of the main body 100. The carabiner, strap, rope, or the like can then be used to attach the container to another piece of equipment for example, by running the attachment mechanism through the another piece of equipment. As an example, a user may use a carabiner to attach the container to a backpack, belt, tent, mode of transportation, or the like.

The main body 100 may be made of plastic, metallic, synthetic, organic, and/or biodegradable materials. In an embodiment the apparatus may be disposable. For example, the apparatus may be used for a period of time and discarded. The apparatus, if made from biodegradable materials may be compostable or degrade in a landfill. As another example, if the container is dropped to an inaccessible location or accidentally forgotten, the container may eventually degrade. Alternatively, an embodiment may use a more durable material. For example, a metallic or plastic material allows a more durable apparatus. The apparatus may be reused after cleaning and returned to use. For example, an outdoor enthusiast may return to an area with proper sewage facilities, empty the contents into a proper sewage receptacle, wash the apparatus, and retain the apparatus for future use on another trip.

In the embodiment shown in the FIGs, but not intended to be limiting in any way, the main body 100 includes two compartments 101A and 101B. The two compartments 101A and 101B may be molded as separate pieces or may be molded as a single piece. In this embodiment, one compartment 101A is larger than the other 101B, with the larger compartment 101A in a substantially vertical orientation. The smaller compartment 101B is angled off the vertical axis by about 20 degrees. Each compartment 101A and 101B has a lid 102A and 102B, respectively. The lid 102A of the larger compartment 101A is about 4.5 inches in diameter, and the lid 102B of the smaller compartment 101B is about 1.64 inches in diameter. A lid 102A and 102B seals a compartment 101A and 101B, respectively. A lid 102A and 102B may have an o-ring or the like to seal the container. The o-ring may be made of a compressable material such as rubber, silicone, soft plastic, or the like.

Each lid 102A and 102B may have a female threaded portion disposed on the inner diameter. The container 101A and 101B may have a male threaded portion adjacent to the open end of the container. The male threaded portion and female threaded portions are complementary. The threaded portion of the lid 102A and 102B and threaded potion of the container 101A and 101B serve to affix the lid to the container in a screw type manner. Each lid 102A and 102B is tethered to the main body 101A and 101B. A tether 103A and 103B may be molded as one piece to a lid at a lid end. The tether 103A and 103B may have a collar structure to fit over the threads on the container at the tether's container end. In an embodiment, the entire product is 4.5 inches wide and 8 inches long. Other sizes may be used to fit the needs of the user. The product may be made of high-density polyethylene (HDPE), plastic, metallic, synthetic, organic, and/or biodegradable materials.

Referring to FIG. 2, an illustration of a side view of an embodiment is shown. The one or more compartments 101A and 101B may be at an angle to one another. In an embodiment, a first compartment 101A is substantially vertical, and the second compartment 101B is angled at or about 20 degrees from vertical. In other embodiments, the one or more compartments may have different varying angles from one another. In an embodiment, the one or more compartments may be parallel or substantially parallel.

Referring to FIG. 3, an illustration of a back view of an embodiment is shown. The main body 101A may contain a smooth surface portion 105. The smooth surface portion 105 may be either recessed or raised from the surface of the main body 101A. The smooth surface portion 105 may be used to affix a label or receive writing from a writing implement. A purpose of the label is such that an individual may write a name or identify the contents on the main body.

Referring to FIG. 4, an illustration of a top view of an embodiment is shown. The top view shows an embodiment of the lid 102A and 102B configuration. A lid 102A and 102B may further comprise an over molded soft touch material to improve grip ability. The lid 102A and 102B may, additionally or alternatively, have indentations, ridges, nubs, cross checking, wing-nut like structures, or the like, 106 to improve grip ability. The gripping structure 106 may be part of the soft overmolding layer or may be part of the underlying plastic structure in which the soft overmolding follows the contours of the gripping structure.

The gripping structures 106 allow a user to open and close a compartment easier. Proper closure of a compartment is necessary because the compartment may contain human waste or items to be protected from the outside environment. The gripping structure 106 may assist outdoor enthusiasts having difficulty opening or closing a lid if hands are wet, dirty, or the like.

An embodiment may apply a soft touch over-molding process. This over molding process may be on any part of the apparatus. This soft touch layer may be laser etched or may be hard tooled. The laser etching through the soft touch layer may be through just a portion of the soft touch layer or the etching may go completely through the soft touch layer to expose the underlying plastic material. Using the soft touch layer etching process the apparatus may be customized. For example, names, logos, trademarks, slogans, artwork, or the like may be etched in the soft touch material. Additionally, different colors may be used for the underlying part and the soft touch overmolding. After etching, the device may have a two-tone appearance.

FIG. 5 illustrates a bottom view of the front and side of an embodiment. This view shows a configuration of elements in an embodiment. In an embodiment the bottom may be convex, concave, or flat. Alternatively, a bottom may have a smaller circumference with respect to the main body 100 such that the apparatus may be placed in a standard cup holder. For example, a cup holder of an automobile or any such holder, rack, pocket, or the like, designed to hold a cup. FIG. 6 illustrates a top view of the front and side of an embodiment. This view shows a configuration of elements in an embodiment. The elements are described in the present disclosure.

As used herein, the singular "a" and "an" may be construed as including the plural "one or more" unless clearly indicated otherwise.

This disclosure has been presented for purposes of illustration and description but is not intended to be exhaustive or limiting. Many modifications and variations will be apparent to those of ordinary skill in the art. The example embodiments were chosen and described in order to explain principles and practical application, and to enable others of ordinary skill in the art to understand the disclosure for various embodiments with various modifications as are suited to the particular use contemplated.

## Claims

**1.** A portable containment apparatus, comprising:
a main body (100);
the main body (100) comprising one or more sealable containers (101A and 101B), each of the one or more sealable containers (101A and 101B) having an inner diameter;
the main body (100) comprising one or more inner walls segregating the one or more sealable containers (101A and 101B) from each of the other one or more sealable containers (101A and 101B); and
one or more lids (102A and 102B) for each of the one of more sealable containers (101A and 101B), wherein the one or more lids (102A and 102B) seal the corresponding one or more sealable containers (101A and 101B).

**2.** The portable containment apparatus of claim 1, wherein at least one of the one or more sealable containers (101A and 101B) are aligned substantially parallel to at least another of the one or more sealable containers (101A and 101B).

**3.** The portable containment apparatus of claim 1, wherein at least one of the one or more sealable containers (101A and 101B) are aligned at substantially an angle to at least another of the one or more sealable containers (101A and 101B).

**4.** The portable containment apparatus of claim 1, wherein the main body (100) is molded as a single unit from high density polyethylene.

**5.** The portable containment apparatus of claim 1, wherein the main body (100) comprises a biodegradable material.

**6.** The portable containment apparatus of claim 1, wherein at least one of the one or more lids (102A and 102B) comprise one or more of indentations, ridges, nubs, cross checking, and wing-nut structures (106).

**7.** The portable containment apparatus of claim 1, wherein at least one of the one or more lids (102A and 102B) comprise one or more layers and wherein at least one of the one or more layers comprises an over molded material.

**8.** The portable containment apparatus of claim 7, wherein the over molded material comprises a laser etched over molded material.

**7.** The portable containment apparatus of claim 1, wherein the one or more sealable containers (101A and 101B) have a different inner diameter.

**8.** The portable containment apparatus of claim 1, wherein the one or more lids (102A and 102B) are connectable to the one or more sealable containers (101A and 101B) via a threaded portion of the one or more lids corresponding to a threaded portion of the corresponding one or more sealable containers.

**9.** The portable containment apparatus of claim 1, wherein the one or more lids (102A and 102B) each comprise a tether (103A and 103B) connectable to the corresponding one or more sealable containers (101A and 101B).

**10.** The portable containment apparatus of claim 1, further comprising one or more tethers (103A and 103B), wherein the one or more tethers (103A and 103B) are connectable between the one or more sealable containers (101A and 101B) and the corresponding one or more lids (102A and 102B).

**11.** The portable containment apparatus of claim 1, wherein the main body (100) further comprises one or more handles (104).

**12.** The portable containment apparatus of claim 1, further comprising at least one o-ring, wherein the at least one o-ring is disposed between the one or more sealable containers (101A and 101B) and the corresponding one or more lids (102A and 102B) when the corresponding one or more lids (102A and 102B) seal the corresponding one or more sealable containers (101A and 101B).

**13.** The portable containment apparatus of claim 1, wherein the main body (100) further comprises one or more recessed areas (105).
